# EUROPEAN PATENT APPLICATION

(11) **EP 4 230 231 A1**
(43) Date of publication of application: **23.08.2023**
(21) Application number: 21886793.5
(22) Date of filing: 27.10.2021
(51) Int. Cl.: A61L 9/20, F24F 8/192

(54) **LIGHT SOURCE MODULE AND AIR CONDITIONING APPARATUS COMPRISING LIGHT SOURCE MODULE**

(30) Priority: 27.10.2020 US 202063106246 P; 15.03.2021 US 202163161228 P
(71) Applicant: Seoul Viosys Co., Ltd., Ansan-si, Gyeonggi-do 15429 (KR)
(72) Inventor: JEONG, Jae Hak, Ansan-Si Gyeonggi-do 15429 (KR); KIM, Ji Won, Ansan-Si Gyeonggi-do 15429 (KR); KIM, Tae Hwa, Ansan-Si Gyeonggi-do 15429 (KR)
(74) Representative: Isarpatent
(86) International application number: PCT/KR2021/015198
(87) International publication number: WO 2022/092799

(57) **Abstract**

A light source module and an air conditioner including the light source module. The light source module includes: at least one light source emitting sterilizing light in a wavelength band capable of sterilizing pollutants; a substrate on which the light source is mounted, the substrate being electrically connected to the light source; and a light guide guiding a traveling direction of the sterilizing light emitted from the light source. The light guide may guide the sterilizing light such that a beam angle of the light source module becomes narrower than a beam angle of the light source.

## Description

### [Technical Field]

Embodiments of the present disclosure relate to a light source module and an air conditioner including the same.

### [Background Art]

Pathogenic infectious agents can be transmitted in the form of droplets or aerosols through air movement. In particular, as the number of transmission cases of the pathogenic infectious agents through air conditioners increases in recent years, there is a need for a technique capable of sterilizing air circulated through the air conditioner.

### [Disclosure]

### [Technical Problem]

Embodiments of the present disclosure provide a light source module capable of sterilizing pollutants in air and an air conditioner including the same.

Embodiments of the present disclosure provide a light source module that allows sterilizing light to be delivered only to a predetermined sterilization area while preventing other components from being exposed to the sterilizing light and an air conditioner including the same.

### [Technical Solution]

In accordance with one aspect of the present disclosure, there is provided a light source module including: at least one light source emitting sterilizing light in a wavelength band capable of sterilizing pollutants; a substrate on which the light source is mounted, the substrate being electrically connected to the light source; and a light guide guiding a traveling direction of the sterilizing light emitted from the light source. The light guide may guide the sterilizing light such that a beam angle of the light source module becomes narrower than a beam angle of the light source.

In accordance with another aspect of the present disclosure, there is provided an air conditioner including: an air inlet through which air is introduced into the air conditioner; an air outlet through which the air is discharged from the air conditioner; an air circulator guiding the air to flow in a direction from the air inlet to the air outlet; a heat exchanger performing heat exchange with the air; and at least one light source module emitting sterilizing light in a wavelength band capable of sterilizing pollutants towards a sterilization area between the air inlet and the heat exchanger. The light source module may include at least one light source emitting the sterilizing light; a substrate on which the light source is mounted, the substrate being electrically connected to the light source; and a light guide guiding a traveling direction of the sterilizing light emitted from the light source. The light guide may guide the sterilizing light to be delivered to the sterilization area such that a beam angle of the light source module becomes narrower than a beam angle of the light source.

### [Advantageous Effects]

The light source module according to the embodiments and the air conditioner including the same can sterilize pollutants in air.

The light source module according to the embodiments and the air conditioner including the same allow sterilizing light to be delivered only to a predetermined sterilization area while preventing other components from being exposed to the sterilizing light and an air conditioner including the same.

### [Description of Drawings]

FIG. 1 is an exemplary view of a light source module according to a first embodiment of the present disclosure.
FIG. 2 is a cross-sectional view of a light source according to an embodiment of the present disclosure.
FIG. 3 is a schematic view of an air conditioner according to an embodiment of the present disclosure.
FIG. 4 is a schematic view of arrangement of components of the air conditioner according to the embodiment of the present disclosure.
FIG. 5 is an exemplary view of an air conditioner including a light source module according to an embodiment of the present disclosure.
FIG. 6 is a block diagram of one example of an air conditioner including a light source module according to an embodiment of the present disclosure.
FIG. 7 is a flowchart showing operation of the air conditioner of FIG. 6.
FIG. 8 is a perspective view of a light source module according to a second embodiment of the present disclosure.
FIG. 9 is a cross-sectional view taken along line B 1-B2 of FIG. 8.
FIG. 10 is a cross-sectional view of a light source module according to a third embodiment of the present disclosure.
FIG. 11 is an exemplary view of a light source module according to a fourth embodiment of the present disclosure.
FIG. 12 is a block diagram of another embodiment of the air conditioner including the light source module according to the embodiment of the present disclosure.

### [Mode for Invention]

Hereinafter, embodiments of the present disclosure will be described with reference to the accompanying drawings. It should be understood that the embodiments are provided for complete disclosure and thorough understanding of the present disclosure by those skilled in the art. Therefore, the present disclosure is not limited to the following embodiments and may be embodied in different ways. In addition, the drawings may be exaggerated in width, length, and thickness of components for descriptive convenience and clarity only. Like components will be denoted by like reference numerals throughout the specification.

In accordance with one aspect of the present disclosure, there is provided a light source module including: at least one light source emitting sterilizing light in a wavelength band capable of sterilizing pollutants; a substrate on which the light source is mounted, the substrate being electrically connected to the light source; and a light guide guiding a traveling direction of the sterilizing light emitted from the light source. The light guide may guide the sterilizing light such that a beam angle of the light source module becomes narrower than a beam angle of the light source.

In one embodiment, the light guide of the light source module may include a first housing and a second housing connected to each other. The first housing may be connected to the second housing to form a right angle or an acute angle therebetween.

The light source may be disposed on an inner surface of the housing. The inner surface of the housing may be one surface of the first housing and one surface of the second housing connected to each other to form a right angle or an acute angle therebetween.

The sterilizing light may be reflected from the one surface of the first housing and the one surface of the second housing.

A width of the first housing may be different from a width of the second housing. The width of the first housing is a length from one end of the first housing connected to the second housing to the other end thereof. In addition, the width of the second housing is a length from one end of the second housing connected to the first housing to the other end thereof.

In another embodiment, the light guide of the light source module may include: a lens portion disposed on the light source and covering the light source; and a cover portion connected to the lens portion and covering surroundings of the light source.

The light guide may be formed of a silicone material allowing transmission of the sterilizing light therethrough.

The light source module may further include a housing on which the light source and the light guide are mounted. The housing may include a first housing and a second housing connected to each other. The first housing may be connected to the second housing to form a right angle or an acute angle therebetween.

The light source may be disposed on an outer surface of the housing. Here, the outer surface of the housing is an opposite surface to an inner surface of the housing. In addition, the inner surface of the housing is one surface of the first housing and one surface of the second housing connected to each other to form a right angle or an acute angle therebetween.

In another embodiment, the light guide of the light source module may include a cavity open at an upper side thereof. An inner wall of the light guide defining the cavity may be an inclined surface. In addition, the light source may be mounted on the cavity.

The light source module may further include a sealing portion sealing the cavity of the light guide and formed of a material allowing transmission of the sterilizing light therethrough.

The light source module may further include a housing on which the light source and the light guide are mounted. The housing may include a first housing and a second housing connected to each other. The first housing may be connected to the second housing to form a right angle or an acute angle therebetween.

The light source may be disposed on an outer surface of the housing. Here, the outer surface of the housing is an opposite surface to an inner surface of the housing. In addition, the inner surface of the housing is one surface of the first housing and one surface of the second housing connected to each other to form a right angle or an acute angle therebetween.

The light source module may further include a sensor unit detecting a pollution level of a sterilization target or detecting a timing of emitting sterilizing light.

In accordance with another aspect of the present disclosure, there is provided an air conditioner including: an air inlet through which air is introduced into the air conditioner; an air outlet through which the air is discharged from the air conditioner; an air circulator guiding the air to flow in a direction from the air inlet to the air outlet; a heat exchanger performing heat exchange with the air; and at least one light source module emitting sterilizing light in a wavelength band capable of sterilizing pollutants towards a sterilization area between the air inlet and the heat exchanger. The light source module may include at least one light source emitting the sterilizing light; a substrate on which the light source is mounted, the substrate being electrically connected to the light source; and a light guide guiding a traveling direction of the sterilizing light emitted from the light source. The light guide may guide the sterilizing light to be delivered to the sterilization area such that a beam angle of the light source module becomes narrower than a beam angle of the light source.

In one embodiment, the light guide of the light source module may include a first housing and a second housing connected to each other. The first housing may be connected to the second housing to form a right angle or an acute angle therebetween.

The light source may be disposed on an inner surface of the housing. The inner surface of the housing may be one surface of the first housing and one surface of the second housing connected to each other to form a right angle or an acute angle therebetween.

The sterilizing light may be reflected from the one surface of the first housing and the one surface of the second housing.

A width of the first housing may be different from a width of the second housing. The width of the first housing is a length from one end of the first housing connected to the second housing to the other end thereof. In addition, the width of the second housing is a length from one end of the second housing connected to the first housing to the other end thereof.

In another embodiment, the light guide of the light source module may include: a lens portion disposed on the light source and covering the light source; and a cover portion connected to the lens portion and covering surroundings of the light source.

The light guide may be formed of a silicone material allowing transmission of the sterilizing light therethrough.

The light source module may further include a housing on which the light source and the light guide are mounted.

The housing may include a first housing and a second housing connected to each other. The first housing may be connected to the second housing to form a right angle or an acute angle therebetween.

The light source may be disposed on an outer surface of the housing. Here, the outer surface of the housing is an opposite surface to an inner surface of the housing. In addition, the inner surface of the housing is one surface of the first housing and one surface of the second housing connected to each other to form a right angle or an acute angle therebetween.

In another embodiment, the light guide of the light source module may include a cavity open at an upper side thereof. An inner wall of the light guide defining the cavity may be an inclined surface. In addition, the light source may be mounted on the cavity.

The light source module may further include a sealing portion sealing the cavity of the light guide and formed of a material allowing transmission of the sterilizing light therethrough.

The light source module may further include a housing on which the light source and the light guide are mounted. The housing may include a first housing and a second housing connected to each other. The first housing may be connected to the second housing to form a right angle or an acute angle therebetween.

The light source may be disposed on an outer surface of the housing. Here, the outer surface of the housing is an opposite surface to an inner surface of the housing. In addition, the inner surface of the housing is one surface of the first housing and one surface of the second housing connected to each other to form a right angle or an acute angle therebetween.

In a further embodiment, the air circulator may be disposed between the air inlet and the heat exchanger. Here, the sterilization area may be a region between the air inlet and the air circulator through which the air passes. In addition, the light source module may be disposed outside the sterilization area to be closer to the air circulator than to the air inlet.

The air inlet and the air circulator may not be exposed to the sterilizing light emitted from the light source module.

In yet another embodiment, the heat exchanger may be disposed between the air inlet and the air circulator. Here, the sterilization area may be a region between the air inlet and the heat exchanger through which the air passes.

For example, the heat exchanger may be an evaporator. Here, the light source module may emit the sterilizing light towards the sterilization area and the evaporator. The air circulator may not be exposed to the sterilizing light.

The air conditioner may further include a sensor unit detecting a pollution level of a sterilization target or detecting a timing of emitting sterilizing light.

Hereinafter, a light source module according to the present disclosure and an air conditioner including the light source module will be described in detail with reference to the accompanying drawings.

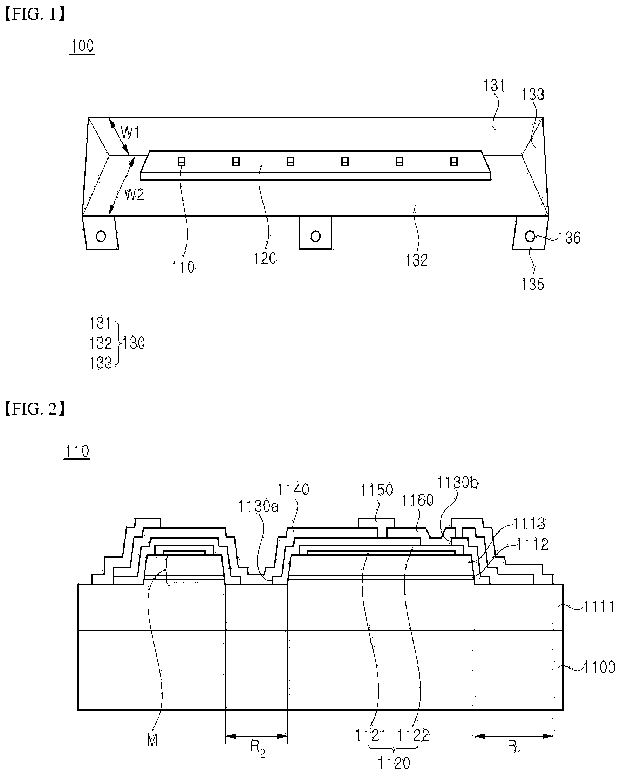

FIG. 1 is an exemplary view of a light source module according to a first embodiment of the present disclosure.

Referring to FIG. 1, a light source module 100 according to the first embodiment may include light sources 110, a substrate 120, and a housing 130.

The light sources 110 may emit sterilizing light in a wavelength band having a sterilization function. For example, the light source 110 may be realized by a light emitting diode chip.

For example, the light source 110 may be a UV light emitting diode chip that emits UV light. The light source 110 may be formed to emit UV light effective for sterilization among UVA, UVB and UVC according to the kind of sterilization target. UV light or sterilizing light in a wavelength band near the UV wavelength band can sterilize a sterilization target through destruction of DNA of pollutants included in the sterilization target. For example, the sterilization target may include water, air, an object, and the like. In addition, the pollutants may include microorganisms, such as bacteria, yeast, mold, viruses, and the like included or buried in the sterilization target.

The light source module 100 may include multiple light sources 110, in which at least some light sources 110 may emit sterilizing light in different wavelength bands.

Accordingly, the light source module 100 may simultaneously emit sterilizing light in various wavelength bands. In addition, the light source module 100 may emit sterilizing light in a wavelength band effective for sterilization according to the kind of sterilization target.

As such, the light source module 100 may emit various combinations of sterilizing light in different wavelength bands in consideration of various factors, such as the kind of sterilization target, the purpose of sterilization, and the like.

FIG. 2 is a cross-sectional view of a light source 110 according to an embodiment of the present disclosure.

Referring to FIG. 2, the light source 110 according to the embodiment may include a growth substrate 1100, a mesa M, a first insulating layer 1130, a first electrode 1140, a second electrode 1120, and a second insulating layer 1150. The mesa M may include a first conductivity type semiconductor layer 1111, an active layer 1112, and a second conductivity type semiconductor layer 1113.

The growth substrate 1100 may be selected from among any substrates enabling growth of the first conductivity type semiconductor layer 1111, the active layer 1112, and the second conductivity type semiconductor layer 1113 thereon. For example, the growth substrate 1100 may be a sapphire substrate, a silicon carbide substrate, a gallium nitride substrate, an aluminum nitride substrate, a silicon substrate, or the like. The growth substrate 1100 may include an inclined side surface. As the growth substrate 1100 includes the inclined side surface, extraction of light generated in the active layer 1112 can be improved.

The first conductivity type semiconductor layer 1111 may be formed on the growth substrate 1100 and the second conductivity type semiconductor layer 1113 may be formed on the first conductivity type semiconductor layer 1111. In addition, the active layer 1112 may be formed between the first conductivity type semiconductor layer 1111 and the second conductivity type semiconductor layer 1113. The first conductivity type semiconductor layer 1111, the active layer 1112, and the second conductivity type semiconductor layer 1113 may include group III-V-based compound semiconductors. For example, the first conductivity type semiconductor layer 1111, the active layer 1112, and the second conductivity type semiconductor layer 1113 may include nitride semiconductors, such as (Al, Ga, In)N.

The first conductivity type semiconductor layer 1111 may include an n-type impurity (for example, Si) and the second conductivity type semiconductor layer 1113 may include a p-type impurity (for example, Mg), or vice versa. The active layer 1112 may include a multi-quantum well structure (MQM).

When forward bias is applied to the light source 110, light is emitted from the active layer 1112 through recombination of electrons and holes therein.

The first conductivity type semiconductor layer 1111, the active layer 1112, and the second conductivity type semiconductor layer 1113 may be grown on the growth substrate 1100 through metal organic chemical vapor deposition (MOCVD) or molecular beam epitaxy (MBE).

The light source 110 may include at least one mesa M. The mesa M may include a plurality of protrusions, which may be spaced apart from each other. However, it should be understood that the structure of the light source 110 is not limited thereto and the light source 110 may include a plurality of mesas (M) spaced apart from each other. The mesa M may be formed to have an inclined side surface by a technique, such as photoresist reflow. The inclined side surface of the mesa M may improve luminous efficiency of the active layer 112.

The first conductivity type semiconductor layer 1111 may include a first contact region R1 and a second contact region R2 exposed through the mesa M.

Since the mesa M is formed through removal of the active layer 1112 and the second conductivity type semiconductor layer 1113 disposed on the first conductivity type semiconductor layer 1111, a region excluding the mesa M becomes the contact regions on an exposed upper surface of the first conductivity type semiconductor layer 1111.

The first electrode 1140 may be electrically connected to the first conductivity type semiconductor layer 1111 through contact with the first contact region R1 and the second contact region R2.

The first contact region R1 may be disposed around the mesa M along an outer periphery of the first conductivity type semiconductor layer 1111. Specifically, the first contact region R1 may be disposed along an outer periphery of the upper surface of the first conductivity type semiconductor layer 1111 between the mesa M and a side surface of the light source 110.

The second contact region R2 may be at least partially surrounded by the mesa M.

A length of the second contact region R2 in a major axis direction thereof may be 0.5 or more times a length of one side of the light source 110.

In this structure, a contact area between the first electrode 1140 and the first conductivity type semiconductor layer 1111 may be increased to allow more effective spreading of electric current flowing from the first electrode 1140 to the first conductivity type semiconductor layer 1111, thereby further reducing forward voltage.

The second electrode 1120 may be disposed on the second conductivity type semiconductor layer 1113 to be electrically connected to the second conductivity type semiconductor layer 1113. The second electrode 1120 may be formed on the mesa M and may have the same shape as the mesa M.

The second electrode 1120 may include a reflective metal layer 1121 and may further include a barrier metal layer 1122.

The barrier metal layer 1122 may cover upper and side surfaces of the reflective metal layer 1121. For example, the barrier metal layer 1122 may be formed to cover the upper and side surfaces of the reflective metal layer 1121 by forming a pattern of the reflective metal layer 1121, followed by forming the barrier metal layer 1122 thereon. For example, the reflective metal layer 1121 may be formed by depositing and patterning Ag, Ag alloy, Ni/Ag, NiZn/Ag, and TiO/Ag layers.

In addition, the barrier metal layer 1122 may be formed of Ni, Cr, Ti, Pt, Au, or a combination thereof. Specifically, the barrier metal layer 1122 may be a combined layer of Ni/Ag/[Ni/Ti]₂/Au/Ti layers sequentially formed on an upper surface of the second conductivity type semiconductor layer 1113.

At least part of an upper surface of the second electrode 1120 may include a 300Å thick Ti layer. When a contact region between the upper surface of the second electrode 1120 and the first insulating layer 1130 is composed of the Ti layer, adhesion between the first insulating layer 1130 and the second electrode 1120 is improved, thereby improving reliability of the light source 110.

An electrode-protecting layer 1160 may be disposed on the second electrode 1120. The electrode-protecting layer 1160 may be formed of the same material as the first electrode 1140, but is not limited thereto.

The first insulating layer 1130 may be disposed between the first electrode 1140 and the mesa M. The first insulating layer 1130 may insulate the first electrode 1140 from the mesa M and may insulate the first electrode 1140 from the second electrode 1120.

The first insulating layer 1130 may partially expose the first contact region R1 and the second contact region R2. Specifically, the first insulating layer 1130 may partially expose the second contact region R2 through an opening 1130a. In addition, the first insulating layer 1130 may cover some region of the first contact region R1 between the outer periphery of the first conductivity type semiconductor layer 1111 and the mesa M such that at least part of the first contact region R1 can be exposed.

The first insulating layer 1130 may be disposed on the second contact region R2 along an outer periphery of the second contact region R2. In addition, the first insulating layer 1130 may be disposed closer to the mesa M than a contact region between the first contact region R1 and the first electrode 1140.

The first insulating layer 1130 may have an opening 1130b that exposes the second electrode 1120. The second electrode 1120 may be electrically connected to a pad or a bump through the opening 1130b.

A contact region between the first contact region R1 and the first electrode 140 is disposed along the entire outer periphery of the upper surface of the first conductivity type semiconductor layer 1111. Specifically, the contact region between the first contact region R1 and the first electrode 1140 may be disposed close to four sides of the first conductivity type semiconductor layer 1111 and may completely surround the mesa M. In this structure, a contact area between the first electrode 1140 and the first conductivity type semiconductor layer 1111 can be increased to allow more efficient spreading of electric current flowing from the first electrode 1140 to the first conductivity type semiconductor layer 1111, thereby further reducing forward voltage.

In one embodiment, the first electrode 1140 and the second electrode 1120 of the light source 110 may be mounted on the substrate 120 directly or via a pad.

For example, when the light source 110 is mounted on the substrate 120 via the pad, two pads (not shown) may be disposed between the light source 110 and the substrate 120. The two pads may adjoin the first electrode 1140 and the second electrode 1120, respectively. For example, the pads may be solders or eutectic metals, without being limited thereto. For example, the eutectic metals may be AuSn.

In another example, when the light source 110 is directly mounted on the substrate 120, the first electrode 1140 and the second electrode 1120 of the light source 110 may be directly bonded to a circuit on the substrate 120. In this case, a bonding material may include a bonding material having electrical conductivity. For example, the bonding material may include at least one conductive material selected from among silver (Ag), tin (Sn), and copper (Cu). However, it should be understood that these are provided by way of example and the bonding material may include various materials having electrical conductivity.

Referring again to FIG. 1, the light source 110 may be mounted on the substrate 120 and may be electrically connected to the substrate 120.

For example, the substrate 120 may be a printed circuit board having a circuit formed thereon.

The substrate 120 may control operation of the light source 110 by supplying electric power to the light source 110 or by stopping power supply thereto.

In this embodiment, the substrate 120 delivers electric power sent from an external device to the light source 110. However, when the light source 110 is directly connected to an external device to receive electric power therefrom, the substrate 120 may be omitted.

The substrate 120 having the light source 110 mounted thereon is mounted on the housing 130.

In this embodiment, the housing 130 is a light guide controlling a direction of sterilizing light emitted from the light source 110.

The housing 130 may include a first housing 131 and a second housing 132.

One end of the first housing 131 is connected to one end of the second housing 132. Here, the first housing 131 may be connected to the second housing 132 to have a certain angle therebetween.

Referring to FIG. 1, one end of the first housing 131 may be connected to one end of the second housing 132 in a longitudinal direction thereof to form a right angle or an acute angle therebetween.

The light sources 110 may be mounted on one of the first housing 131 and the second housing 132.

According to this embodiment, the substrate 120 having the light source 110 mounted thereon may be mounted on the second housing 132. Here, the substrate 120 may be mounted on one surface of the second housing 132 connected to the first housing 131 to form an acute angle therebetween.

Since the first housing 131 is connected to the second housing 132 to form a right angle or an acute angle therebetween, the sterilizing light emitted from the light source 110 may be intensively delivered to a predetermined region by the first housing 131 or the second housing 132.

The area and angle of the first housing 131 and the second housing 132 may be changed in consideration of a beam angle of the light source 110, an delivery range of the sterilizing light, and the like.

Although the substrate 120 having the light sources 110 mounted thereon is disposed on one surface of the second housing 132 in this embodiment, it should be understood that the locations of the light sources 110 are not limited thereto. The light sources 110 may be disposed on one surface of the first housing 131, which is connected to the second housing 132 to form an acute angle therebetween, depending upon a traveling direction of the sterilizing light.

The housing 130 may be formed of a material reflecting the sterilizing light or preventing transmission of the sterilizing light therethrough.

For example, the housing 130 may be formed of a polymer resin that does not allow transmission of light therethrough. Accordingly, the housing 130 can prevent the sterilizing light emitted from the light source 110 from being emitted to a region other than a predetermined region. That is, the housing 130 may allow only irradiation of a predetermined region with the sterilizing light.

Alternatively, the housing 130 may be formed of a metal that reflects the sterilizing light. For example, the housing 130 may be formed of aluminum, stainless steel, and the like.

Accordingly, light emitted from the light source 110 may be reflected from the housing 130 to be delivered to a predetermined region.

Since the sterilizing light is reflected from the housing 130 instead of being absorbed thereto, the housing 130 formed of the metal may reduce loss of the sterilizing light. Accordingly, the housing 130 may increase the quantity of light delivered to a predetermined region, thereby improving sterilization efficiency.

As such, sterilizing light emitted from the light source 110 may be intensively delivered to a predetermined region by the housing 130 acting as the light guide. That is, the housing 130 acting as the light guide may make a beam angle of sterilizing light emitted from the light source module 100 narrower than a beam angle of sterilizing light emitted from the light source 110. Here, the beam angle of the light source 110 corresponds to the beam angle of the sterilizing light on the surface of the light source 110. In addition, the beam angle of the light source module 100 corresponds to the beam angle of the sterilizing light on a boundary line between an interior and an exterior of the housing 130.

Referring to FIG. 1, a width W1 of the first housing 131 may be different from a width W2 of the second housing 132. Here, the width W1 of the first housing 131 is a length from one end of the first housing 131 connected to the second housing 132 to the other end thereof. In addition, the width W2 of the second housing 132 is a length from one end of the second housing 132 connected to the first housing 131 to the other end thereof. The other end of the first housing 131 refers to an end of the first housing 131 placed in an opposite direction to one end of the first housing 131 and the other end of the second housing 132 refers to an end of the second housing 132 placed in an opposite direction to one end of the second housing 132.

The first housing 131 and the second housing 131 having different widths may be placed by taking into account a predetermined traveling direction of sterilizing light and a region required to prevent the sterilizing light from reaching the region depending on a securing location of the light source module or a sterilization target (or sterilization area). Here, it may be determined where the light sources 110 and the substrate 120 are to be placed on the first housing 131 and the second housing 132.

That is, according to this embodiment, the light source module 100 allows the sterilizing light to be intensively delivered to the sterilization target (or sterilization area) as much as possible through change in placement of the first housing 131, the second housing 132 and the light sources 110 according to the location of the light source module 100 and the direction in which the sterilizing light is delivered.

However, the width W1 of the first housing 131 is not necessarily different from the width W2 of the second housing 132 but may be the same as the width W2.

Referring to FIG. 1, the housing 130 may include side surfaces 133 connected to opposite sides of the first housing 131 and opposite sides of the second housing 132, which are separated from the opposite sides of the first housing 131, respectively. Each of the side surfaces of the housing 130 connects the separated sides of the first housing 131 and the second housing 132 to each other to prevent light emitted from the light source 110 from escaping through opposite sides of the housing 130. Here, the opposite sides of the housing 130 refer to opposite ends of the housing in the longitudinal direction thereof, respectively.

The housing 130 formed of a metal may improve heat dissipation efficiency of the light source module 100.

Alternatively, the housing 130 may have an inner surface coated with a light reflective material.

The inner surface of the housing 130 refers to one surface of the first housing 131 and one surface of the second housing 132 connected to each other to form an acute angle therebetween.

The first housing 131 may be connected to the second housing 132 to allow adjustment in angle therebetween. For example, the first housing 131 may be connected to the second housing 132 by a bendable material. Alternatively, the first housing 131 may be connected to the second housing 132 by a hinge. In this way, the first housing 131 and the second housing 132 may be connected to each other to allow adjustment in angle in various ways.

The housing 130 may further include a fastening portion 135 that secures the light source module 100 at a predetermined location.

Referring to FIG. 1, the fastening portion 135 may be formed on the other end of the second housing 132. The fastening portion 135 may be formed with a coupling hole to be coupled to a screw.

For example, with the fastening portion 135 brought into contact with a certain portion of an external device, the light source module 100 may be secured to the external device through the fastening portion using the screw.

In addition, the fastening portion 135 allows adjustment in angle of the light source module 100 through change of a connection angle to the second housing 132. For example, the fastening portion 135 may include a bendable soft portion or may include a hinge structure.

Although the fastening portion 135 is provided to the second housing 132 and has a screw fastening structure in this embodiment, it should be understood that the fastening portion 135 is not limited thereto.

The fastening portion 135 may be formed at various locations on the housing 130 in consideration of the structure of an external device to which the light source module 100 is secured and the delivery direction of the sterilizing light, and may have various structures.

In this embodiment, the fastening portion 135 is fastened to the external device such that the light source module 100 can be secured to the external device. Alternatively, the first housing 131 or the second housing 132 of the housing 130 may be directly fastened to the external device. For example, the first housing 131 or the second housing 132 may be secured to the external device via a bonding material. Alternatively, the first housing 131 or the second housing 132 may be formed with a fastening hole 136, through which the first housing 131 or the second housing 132 is fastened to the external device by a screw.

In the structure wherein the first housing 131 or the second housing 132 is directly secured to the external device through close contact therebetween, the fastening portion 135 may be omitted.

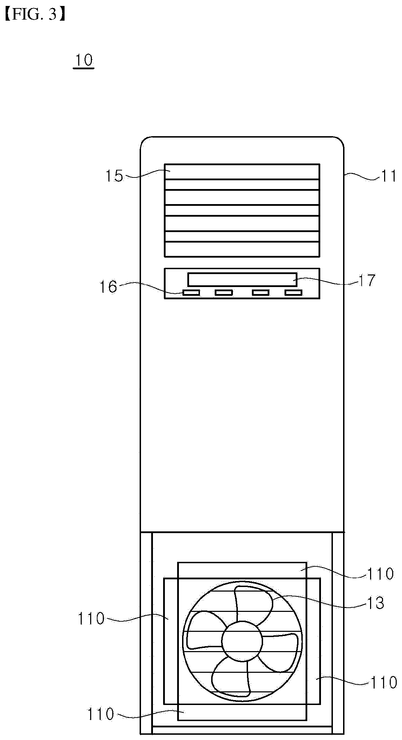

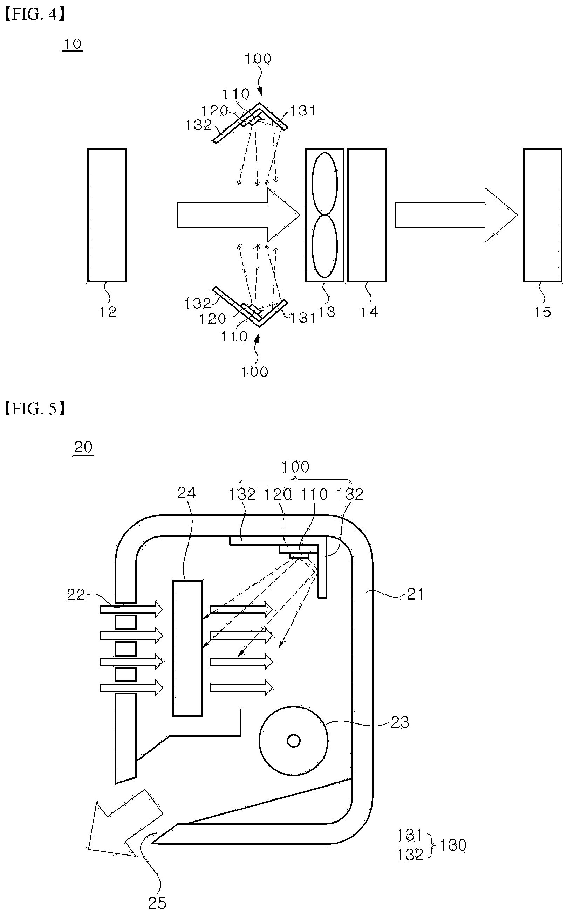

FIG. 3 and FIG. 4 are exemplary views of an air conditioner including a light source module according to an embodiment of the present disclosure.

FIG. 3 is a schematic view of an air conditioner 10 according to an embodiment of the present disclosure. FIG. 4 is a schematic view of arrangement of components of the air conditioner according to the embodiment of the present disclosure.

According to this embodiment, the air conditioner 10 may include a body 11, an air inlet 12, an air outlet 15, an air circulator 13, a heat exchanger 14, and a light source module 100.

The body 11 is provided with the air inlet 12 and the air outlet 15.

The air conditioner 10 according to this embodiment has a structure in which the air inlet 12 is disposed at a lower portion of the body 11 and the air outlet 15 is disposed at an upper portion of the body 11. In FIG. 3, in order to show the air circulator 13 and the light source module 100 inside the body 11, a portion of the body 11 formed with the air inlet 12 is omitted. Although not shown in FIG. 3, the air inlet 12 may be formed at a location facing the air circulator 13.

The air circulator 13, the heat exchanger 14 and the light source module 100 may be disposed inside the body 11. The light source module 100 is the light source module 100 according to the first embodiment shown in FIG. 1.

Although not shown in the drawings, the body 11 of the air conditioner 10 may be provided with at least one kind of filter to filter out pollutants from air. For example, the filter may include at least one selected from among a pre-filter, a HEPA filter, a carbon filter, and a photocatalytic filter. The pre-filter and the HEPA filter may separate pollutants from air. In addition, the carbon filter and the photocatalytic filter may perform removal of pollutants and deodorization. When the photocatalytic filter is disposed in the air conditioner 10, the air conditioner 10 may be further provided with a light source 110 for promoting photocatalytic reaction. Alternatively, the photocatalytic filter may be disposed in a region to be irradiated with sterilizing light.

The air circulator 13 may form an air flow such that external air can be introduced into the body 11 and then can be discharged through the air outlet 15 after the air is subjected to heat exchange. For example, the air circulator 13 may be a fan that forms an air stream.

The heat exchanger 14 transfers heat from a relatively hot object to a relatively cold object. That is, the heat exchanger 14 can transfer heat to air introduced through the air inlet 12 of the body 11 or can recover heat from the air. For example, the heat exchanger 14 may include at least one of an evaporator, a condenser, and a cooler.

When the air conditioner 10 is a cooling device, the heat exchanger 14 may recover heat from air. In addition, when the air conditioner 10 is a heating device, the heat exchanger may heat air.

Referring to FIG. 4, the air conditioner 10 according to this embodiment may have a sequential arrangement of the air inlet 12, the light source module 100, the air circulator 13, the heat exchanger 14, and the air outlet 15 corresponding to a flow direction of air. That is, the light source module 100 may be disposed between the air inlet 12 and the air circulator 13.

Referring to FIG. 3 and FIG. 4, the light source module 100 may be placed in a direction perpendicular to an air flow direction.

That is, the light source module 100 may be disposed around a space in which the air flows, and may emit light toward the air passing through a space between the air inlet 12 and the air circulator 13. Although not shown in the drawings, the light source module 100 may be secured to an inner space of the body 11 of the air conditioner 10 by the fastening portion 135 (see FIG. 1).

In this embodiment, since the light source module 100 does not obstruct the air flow, it is possible to prevent decrease in area of the path through which the air passes.

Referring to FIG. 4, the light source module 100 may be disposed in consideration of the traveling direction of light and a region through which the air passes.

The light source 110 generally has a beam angle of about 120 degrees.

Accordingly, when the housing 130 is omitted, the sterilizing light emitted from the light source 110 spreads broadly and reaches the air circulator 13 near the light source 110. In addition, the sterilizing light emitted from the light source 110 may spread broadly and reach an external space of the body 11 through the air inlet 12.

When the air circulator 13 is irradiated with sterilizing light in the UV wavelength band or in a wavelength band near the UV wavelength band, the air circulator 13 can suffer from discoloration or deterioration in properties.

When exposed to the sterilizing light discharged from the body 11, a human body can be damaged by the sterilizing light.

The housing 130 may limit a traveling direction and a delivery region of the sterilizing light emitted from the light source 110.

Referring to FIG. 4, the light source module 100 may be disposed to be slightly oblique towards the air circulator 13 instead of being disposed to face the sterilization area. Here, the sterilization area is a space between the air inlet 12 and the air circulator 13, through which air passes. That is, referring to FIG. 3, multiple light source modules 100 may be disposed to surround the sterilization space. That is, the multiple light source modules 100 may surround the sterilization space such that the sterilization space can be intensively irradiated with the sterilizing light.

Referring to FIG. 3, the air conditioner 10 is provided with four light source modules 100. However, it should be understood that the number of light source modules 100 is not limited thereto and may be changed in various ways.

The light source module 100 is obliquely disposed in consideration of the beam angle of the light source 110, thereby preventing the sterilizing light from leaking to the outside of the body 11 through the air inlet 12. Further, even when some of the sterilizing light is discharged in a direction of the air inlet 12, the sterilizing light is reflected from the second housing 132 to travel towards the sterilization area.

Among the sterilizing light emitted from the light source 110, sterilizing light traveling towards the air circulator 13 is reflected from the first housing 131 to travel towards the sterilization area.

In addition, the sterilizing light discharged from the light source 110 and traveling towards the air circulator 13 may be reflected from the first housing 131 to travel towards the sterilization area.

As such, the light source module 100 according to this embodiment includes the housing 130, which acts as the light guide, to prevent the sterilizing light from traveling to the outside of the body 11 and other components, thereby preventing a human body and other components of the air conditioner 10 from being damaged by the sterilizing light.

Further, according to this embodiment, the light source module 100 may prevent the other components of the air conditioner 10 from being exposed to the sterilizing light using the housing 130 and thus may be disposed adjacent to the other components. That is, the light source module 100 may be disposed as far from the air inlet 12 as possible. Accordingly, the light source module 100 according to this embodiment may prevent the sterilizing light from traveling to the outside of the body 11 using the housing 130.

Further, according to this embodiment, the housing 130 of the light source module 100 may maximize the quantity of light delivered to the sterilization area. In addition, according to this embodiment, the housing 130 of the light source module 100 may maximize the sterilization area while preventing the outside of the body 11 and other components from being exposed to the sterilizing light. Accordingly, in the light source module 100 according to this embodiment, the housing 130 allows air flowing to the air circulator 13 through the air inlet 12 to be exposed to the sterilizing light as much as possible, thereby improving air sterilization efficiency.

Further, since the light source module 100 according to this embodiment allows easy adjustment of a region to be irradiated with light, the light source module 100 allows the sterilizing light to be intensively delivered to a predetermined sterilization area even when disposed at any place in a space where air flows. Thus, the light source module 100 according to this embodiment may be provided to any kind of air conditioner 10 even without a separate installation space, regardless of the structure of the air conditioner 10.

In addition, the light source module 100 according to this embodiment may sterilize small pollutants that cannot be filtered out by a filter disposed inside the air conditioner 10, thereby improving air sterilization effect.

The air conditioner 10 may further include an input unit 16 to manipulate operation of the air conditioner 10 and a display unit 17 to monitor operation of the air conditioner 10. Further, although not shown in the drawings, the air conditioner 10 may further include a controller that controls operation of each component of the air conditioner 10 in response to a signal received through the input unit 16.

FIG. 5 is an exemplary view of an air conditioner including a light source module according to an embodiment of the present disclosure.

Referring to FIG. 5, an air conditioner 20 according to this embodiment may include a body 21, an air inlet 22, an air outlet 25, an air circulator 23, a heat exchanger 24, and a light source module 100.

In the air conditioner 20 according to this embodiment, the body 21, the air inlet 22, the air outlet 25, the air circulator 23, the heat exchanger 24 and the light source module 100 have substantially the same functions as the body 11 of the air conditioner 10, the air inlet 12, the air outlet 15, the air circulator 13, the heat exchanger 14 and the light source module 100 shown in FIG. 3 and FIG. 4.

Accordingly, the following description will focus on different features of the air conditioner 20 according to this embodiment from those of the air conditioner shown in FIG. 2 and FIG. 4.

In the air conditioner 20 according to this embodiment, the air inlet 22 is placed at an upper portion of the body 21 and the air outlet 25 is placed at a lower portion of the body 21.

The air conditioner 20 according to this embodiment may have a sequential arrangement of the air inlet 22, the heat exchanger 24, the air circulator 23 and the air outlet 25 corresponding to a flow direction of air. In addition, although not shown in the drawings, the body 21 of the air conditioner 20 may be provided with at least one kind of filter to filter out pollutants from air. For example, the filter may be disposed between the air inlet 22 and the heat exchanger 24.

In the air conditioner 20 according to this embodiment, the light source module 100 may be disposed between the heat exchanger 24 and the air circulator 23. Further, the light source module 100 may be secured to an inner wall of the body 21.

In this embodiment, the light source module 100 may emit sterilizing light towards a sterilization area corresponding to a space between the heat exchanger 24 and the air circulator 23.

Referring to FIG. 5, the light emitted from the light source 110 and traveling towards the air circulator 23 may be reflected from the first housing 131 to be delivered to the sterilization area.

Accordingly, the light source module 100 may prevent the air circulator 23 from being exposed to and damaged by the sterilizing light using the housing 130.

For example, when the heat exchanger 24 is an evaporator, the light source module 100 may emit sterilizing light towards the heat exchanger 24. When the heat exchanger 24 is the evaporator, the heat exchanger 24 may reduce the temperature of air through absorption of heat from the air introduced into the body 21 through the air inlet 22. In this case, moisture in the air forms water droplets on a surface of the heat exchanger 24, thereby increasing the possibility of mold generation or pollution by pollutants.

The air conditioner 20 according to this embodiment may be disposed to allow the light source module 100 to emit sterilizing light not only towards the sterilization area but also towards the heat exchanger 24 provided as the evaporator.

Referring to FIG. 5, the light source module 100 may be secured to the interior of the air conditioner 20 such that the second housing 132 closely contacts an inner wall of the body 21. Thus, since the second housing 132 is not placed between the light source 110 and the heat exchanger 24, some of the sterilizing light emitted from the light source 110 may reach the heat exchanger 24.

Accordingly, the light source module 100 may emit sterilizing light towards the surface of the evaporator to prevent mold generation or pollution that can occur in a humid environment or to sterilize mold or pollution that has already occurred in the evaporator.

As such, the light source module 100 may emit sterilizing light not only towards air passing through the body 21, but also towards components where pollution can occur. Accordingly, the air conditioner 20 according to this embodiment simultaneously sterilizes air and a component in an easily contaminated environment through the light source module 100 having the housing 130, thereby improving air sterilization efficiency.

The air conditioner 20 according to this embodiment has a structure wherein the second housing 132 of the light source module 100 is secured to an upper inner wall of the body 21. However, the structure of the light source module 100 secured to the interior of the air conditioner 20 may be modified in various ways. That is, depending upon the locations of the sterilization area and components pertaining to a sterilization target, such as an evaporator and the like, an installation location and a securing angle of the light source module 100 may be changed in various ways.

Although not shown in FIG. 5, the air conditioner 20 according to this embodiment may also further include an input unit to manipulate operation of the air conditioner 20, a display unit to monitor operation of the air conditioner 20, and a controller to control operation of components of the air conditioner 20.

In FIG. 3 and FIG. 5, an application example of the light source module 100 (see FIG. 1) according to the first embodiment to the air conditioner having various structures is described.

However, the light source module applied to the air conditioner is not limited to the light source module 100 (see FIG. 1) according to the first embodiment of the present disclosure. It should be understood that light source modules according to various embodiments of the present disclosure described below may also be applied to air conditioners having various structures.

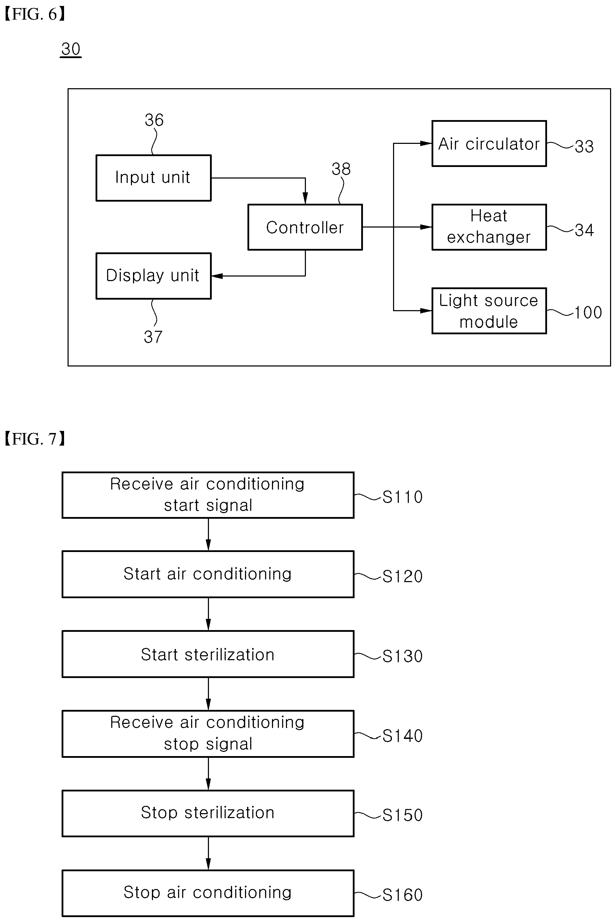

FIG. 6 is a block diagram of one example of an air conditioner including a light source module according to an embodiment of the present disclosure.

Referring to FIG. 6, an air conditioner 30 according to this embodiment includes an input unit 36, a display unit 37, a controller 38, an air circulator 33, a heat exchanger 34, and a light source module 100.

The input unit 36 may generate an air conditioning start signal for operation of the air conditioner 30 or an air conditioning stop signal for stopping operation of the air conditioner 30 in response to a manipulation signal input from outside.

Then, the input unit 36 may send the air conditioning start signal or the air conditioning stop signal to the controller 38. The air conditioning start signal may include information on the type of air conditioning and information on an air conditioning time. Here, the information on the type of air conditioning may include information on cooling, heating, ventilation, and the like.

The controller 38 may control operation of each of components constituting the air conditioner 30 in response to the signal sent from the input unit 36.

The controller 38 may control operation of the air circulator 33 and the heat exchanger 34 according to information included in the air conditioning start signal. In addition, the controller 38 may control operation of the light source module 100 according to information included in the air conditioning start signal.

The controller 38 may control the air circulator 33, the heat exchanger 34 and the light source module 100 to stop air conditioning operation and sterilization operation at a preset time according to the information on the air conditioning time included in the air conditioning start signal.

If the air conditioning start signal does not include the information on the air conditioning time, the controller 38 may control the air circulator 33, the heat exchanger 34 and the light source module 100 so as to stop air conditioning operation and sterilization operation of the air conditioner 30 in response to the air conditioning stop signal sent from the input unit 36.

The display unit 37 may display information on the air conditioning operation and the sterilization operation of the air conditioner 30. As a result, a user can check an operating state of the air conditioner 30 through the display unit 37.

FIG. 7 is a flowchart illustrating operation of the air conditioner shown in FIG. 6.

Referring to FIG. 6 and FIG. 7, first, the controller may receive an air conditioning start signal for operation of the air conditioner 30 through the input unit 36. (S 110)

In response to the air conditioning start signal, the controller 38 may control each of components of the air conditioner 30 to allow the air conditioner 30 to perform air conditioning operation. (S 120)

For example, the air conditioner 30 may operate the air circulator 33 to generate an air flow from the air inlet to the air outlet. In addition, the air conditioner 30 may operate the heat exchanger 34 to perform heat exchange of the air introduced into the air conditioner 30. Here, the controller 38 may not allow operation of the heat exchanger 34 in response to the air conditioning start signal.

When the air conditioner 30 starts the air conditioning operation, the controller 38 may operate the light source module 100 to perform sterilization of air. (S 130)

Here, the controller 38 controls the light source module 100 to allow the air conditioning operation and the sterilization operation to be sequentially performed. Alternatively, the controller 38 may operate the light source module 100 and the air circulator 33 at the same time such that the air conditioning operation and the sterilization operation can be simultaneously performed.

Then, the controller 38 may receive an air conditioning stop signal. (S140)

In response to the air conditioning stop signal, the controller 38 may control each of the components of the air conditioner 30 to stop the air conditioning operation of the air conditioner 30. (S150)

The controller 38 may stop operation of the light source module 100 to stop sterilization of the air in response to the air conditioning stop signal. (S 160)

Here, although the air conditioning operation and the sterilization operation are sequentially stopped, the controller 38 may control the light source module 100 to stop the air conditioning operation and the sterilization operation at the same time.

The controller 38 may stop the air conditioning operation and the sterilization operation of the air conditioner 30 at a preset time based on information on an air conditioning time included in the air conditioning stop signal even without receiving the air conditioning stop signal.

As such, the light source module 100 according to this embodiment may allow sterilization to be automatically performed during operation of the air conditioner 30.

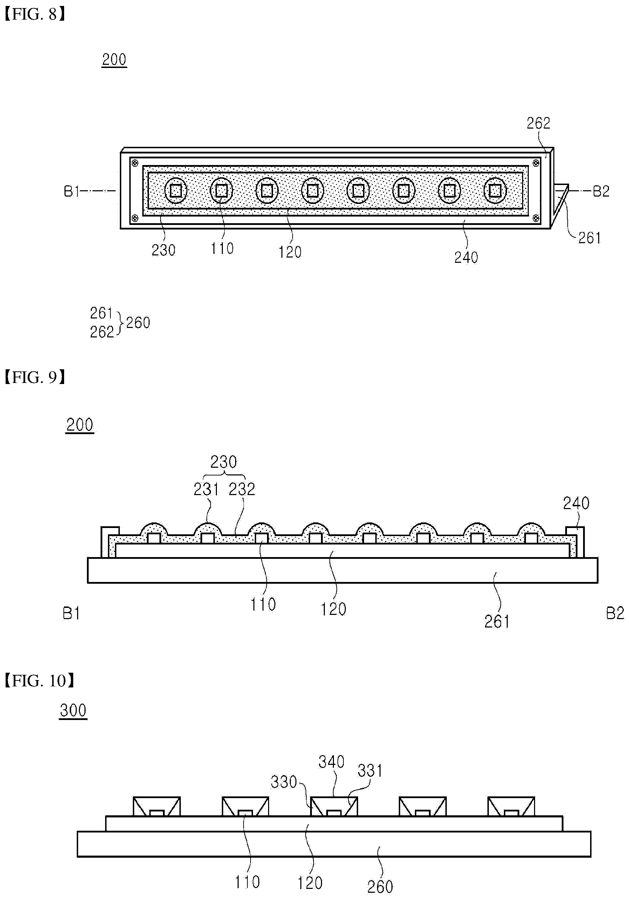

FIG. 8 and FIG. 9 are exemplary views of a light source module according to a second embodiment of the present disclosure. FIG. 8 is a perspective view of the light source module according to the second embodiment. FIG. 9 is a cross-sectional view taken along line B1-B2 of FIG. 8.

A light source module 200 according to the second embodiment may include light sources 110, a substrate 120, a housing 260, light guides 230, and a securing portion 240.

For description of the light sources 110, the substrate 120 and the housing 260 of the light source module 200 according to this embodiment, refer to description of the light source module 100 (see FIG. 1) according to the first embodiment.

The housing includes a first housing 261 and a second housing 262.

The first housing 261 may secure the light source module 200 to an external device. For example, the first housing 261 may be secured to the external device via a bonding material such that a module can be secured thereto. In addition, the first housing 261 may be formed with a fastening hole (not shown). Accordingly, the first housing 261 may be screwed to the external device through a fastening hole such that the light source module 200 can be secured to the external device.

The housing 260 may further include a separate fastening portion as in the housing 130 of the light source module 100 according to the first embodiment (see FIG. 1).

The housing 260 of the light source module 200 according to this embodiment does not include a side surface covering the opposite sides of each of the first housing 261 and the second housing 262. Alternatively, the housing 260 according to this embodiment may also have opposite sides as in the housing 130 of the light source module 100 according to the first embodiment (see FIG. 1).

The substrate 120 and the light sources 110 are mounted on the second housing 262.

In the light source module 100 according to the first embodiment (see FIG. 1), the light sources 110 and the substrate 120 are disposed on one surface of the second housing 262, whereas the light sources 110 and the substrate 120 are disposed on the other surface of the second housing 262 in the light source module 200 according to this embodiment. Here, one surface of the second housing 262 refers to a surface of the second housing connected to the first housing 261 to form an acute angle therebetween, and the other surface of the second housing 262 refers to an opposite surface of the one surface of the second housing 262.

The first housing 261 is secured at the other surface thereof to an external device and a delivery direction of sterilizing light may be changed depending upon an angle between the first housing 261 and the second housing 262.

Referring to FIG. 8 and FIG. 9, the light guide 230 may cover the substrate 120 and the multiple light sources 110 in the second housing 262.

Referring to FIG. 9, the light guide 230 may include a cover portion 232 and a lens portion 231.

The lens portion 231 may be formed to cover the light sources 110.

The lens portion 231 may serve to guide light emitted from the light source 110.

For example, the lens portion 231 may reduce a beam angle of the sterilizing light emitted from the light source 110. That is, the lens portion 231 may reduce a delivery range of the sterilizing light such that the sterilizing light can be intensively delivered to a region within a predetermined range.

Alternatively, the lens portion 231 may change the traveling direction of the sterilizing light emitted from the light source 110. For example, the lens portion 231 allows the sterilizing light emitted from the light source 110 to travel only in a lateral direction of the lens portion 231 or in an upward direction thereof.

As such, the irradiation range and the traveling direction of the sterilizing light may be changed according to the structure of the lens portion 231.

Accordingly, the light source module 200 according to this embodiment may control the traveling direction of the sterilizing light merely by replacing the light guide 230 with the light guide 230 including the lens portion 231 having a different structure.

As such, the light guide 230 of the light source module 200 according to this embodiment may allow the sterilizing light to travel only towards a predetermined sterilization area instead of traveling towards other components.

In addition, the light guide 230 of the light source module 200 according to this embodiment may allow change of a location to be irradiated with the sterilizing light even without changing an installation location of the light source module 200.

According to this embodiment, the lens portion 231 is formed in an upwardly convex dome shape on an upper surface of the light source 110. However, it should be understood that the lens portion 231 is not limited to this structure. The lens portion 231 may be modified in various ways so long as the lens portion 231 can act as the light guide 230 to allow the sterilizing light emitted from the light source 110 to be intensively delivered to a predetermined area.

The cover portion 232 may be formed to cover a region other than a region covered by the lens portion 231.

As such, the light guide 230 may be formed to protect the substrate 120 and the light source 110 from foreign matter such as moisture, dust and the like by covering the entirety of the substrate 120 and the light sources 110.

The light guide 230 may be formed of a material allowing transmission of the sterilizing light therethrough. Alternatively, in the light guide 230, the lens portion 231 may be formed of a material allowing transmission of the sterilizing light therethrough and the cover portion 232 may be formed of a material not allowing transmission of the sterilizing light therethrough.

The light guide 230 may be formed of an elastic polymer material. For example, the light guide 230 may be formed of a silicone resin.

Accordingly, the light guide 230 may exhibit high adhesion to the housing 260, the substrate 120 and the light source 110.

As such, the sterilizing light emitted from the light source 110 may be intensively delivered to a predetermined region by the light guide 230. That is, the light guide 230 may make a beam angle of the sterilizing light emitted from the light source module 200 narrower than a beam angle of sterilizing light emitted from the light source 110. Here, the beam angle of the light source 110 refers to a beam angle of the sterilizing light on a surface of the light source 110. In addition, the beam angle of the light source module 200 refers to a beam angle of the sterilizing light on a surface of the light guide 230.

The securing portion 240 may secure the light guide 230 to the housing 260.

Referring to FIG. 8 and FIG. 9, the securing portion 240 may be formed to cover a periphery of the light guide 230. In addition, the securing portion 240 may be secured to the housing 260 by a screw.

As the securing portion 240 is secured to the housing 260, the light guide 230 may closely contact the housing 260 so as not to be separated from the housing 260, the substrate 120 and the light sources 110.

According to this embodiment, the securing portion 240 is used to secure the light guide 230 to the housing 260. However, when the light guide 230 is directly secured to the housing 260 via a bonding material and the like, the securing portion 240 may be omitted.

In addition, according to this embodiment, the light sources 110, the substrate 120 and the light guides 230 are disposed on the outer surface of the housing 260, but are not limited thereto.

The light sources 110, the substrate 120 and the light guides 230 may be disposed on an inner surface of the housing 260.

Here, the inner surface of the housing 260 refers to a surface of the housing 260 on which the first housing 261 and the second housing 262 meet each other to form a right angle or an acute angle therebetween. That is, the inner surface of the housing 260 includes one surface of the first housing 261 and one surface of the second housing 262. In addition, the outer surface of the housing 260 refers to an opposite surface of the inner surface of the housing 260 and includes the other surface of the first housing 261 and the other surface of the second housing 262.

As such, in the structure in which the light sources 110, the substrate 120 and the light guides 230 are disposed on the inner surface of the housing 260, the light guide 230 may primarily guide the sterilizing light emitted from the light source 110 and the housing 260 may secondarily guide the sterilizing light emitted from the light guide 230. Accordingly, since the light source module guides the sterilizing light twice, the light source module may allow a greater quantity of the sterilizing light to reach the sterilization area.

FIG. 10 is a cross-sectional view of a light source module according to a third embodiment of the present disclosure.

Referring to FIG. 10, a light source module 300 according to the third embodiment may include light sources 110, a substrate 120, a housing 260, light guides 330, and a sealing portion 340.

For description of the light sources 110, the substrate 120 and the housing 260 of the light source module 300 according to this embodiment, refer to description of the light source module 200 (see FIG. 8 and FIG. 9) according to the second embodiment. In addition, the housing 260 shown in FIG. 10 has the same structure as the housing 260 (see FIG. 8 and FIG. 9) according to the second embodiment.

The light guide 330 is formed to surround a side surface of the light source 110. For example, the light guide 330 may include a cavity 331 in which the light source 110 is mounted.

The light guide 330 may include an inclined inner wall that defines the cavity 331.

The beam angle of the sterilizing light emitted from the light source 110 may be changed depending upon an inclination of the inner wall of the light guide 330. For example, as the inclination of the inner wall of the light guide 330 approaches 90 degrees, the beam angle of the sterilizing light emitted from the light guide 330 is narrowed.

Referring to FIG. 10, the light source module 300 includes multiple light sources 110, which may be mounted on multiple light guides 330, respectively. However, the present disclosure is not limited to the structure wherein all of the multiple light sources 110 are mounted on the light guides 330, respectively.

In order to allow the sterilizing light to be intensively delivered to a predetermined sterilization area, the beam angle of the light sources 110 disposed on the periphery of the substrate 120 may be controlled. In this case, the light guide 330 may be provided only to each of the light sources 110 disposed on the periphery of the substrate 120 required to narrow the beam angle.

Alternatively, with the light guides 330 disposed on the light sources 110, respectively, a light guide 330 having a different structure may be provided to the light source 110 on the periphery of the substrate 120. For example, the light guide 330 disposed closer to the periphery of the substrate may be formed to have an inner wall having an inclination closer to 90 degrees than other inner walls of the light guide 330 disposed on the periphery thereof.

As such, the light source module 300 according to this embodiment allows the sterilization area to be intensively irradiated with the sterilizing light through the light guide 330 formed with the cavity 331, which receives the light source 110 therein, while preventing a region other than the sterilization area from being irradiated with the sterilizing light.

In addition, the light guide 330 may be formed of a material reflecting the sterilizing light. Alternatively, the material reflecting the sterilizing light may be coated on the inner wall of the light guide 330 defining the cavity 331.

Accordingly, the sterilizing light emitted from the light source 110 may be reflected from the inner wall of the light guide 330 to be discharged in an upward direction of the light sources 110.

In the light source module 300 according to this embodiment, the light guide 330 is formed to reflect the sterilizing light, thereby improving sterilization efficiency through increase in quantity of the sterilizing light delivered to the sterilization area.

The cavity 331 of the light guide 330 is formed with the sealing portion 340.

The sealing portion 340 fills the cavity 331 of the light guide 330 and may be formed of a material allowing transmission of the sterilizing light therethrough. For example, the sealing portion 340 may be formed of an epoxy resin. However, it should be understood that the sealing portion 340 is not limited thereto and may be formed of any material capable of filling the cavity 331 of the light guide 330 while allowing transmission of the sterilizing light therethrough.

Although the sealing portion 340 fills the cavity 331 of the light guide 330 in FIG. 10, it should be understood that the material and structure of the sealing portion 340 are not limited thereto. For example, the sealing portion 340 may have a plate structure allowing transmission of the sterilizing light therethrough. That is, the sealing portion 340 may be a transmission window that covers the cavity 331.

In the light source module 300 according to this embodiment, the cavity 331 of the light guide 330 is sealed by the sealing portion 340 to protect the light source 110 from foreign matter, such as dust and moisture.

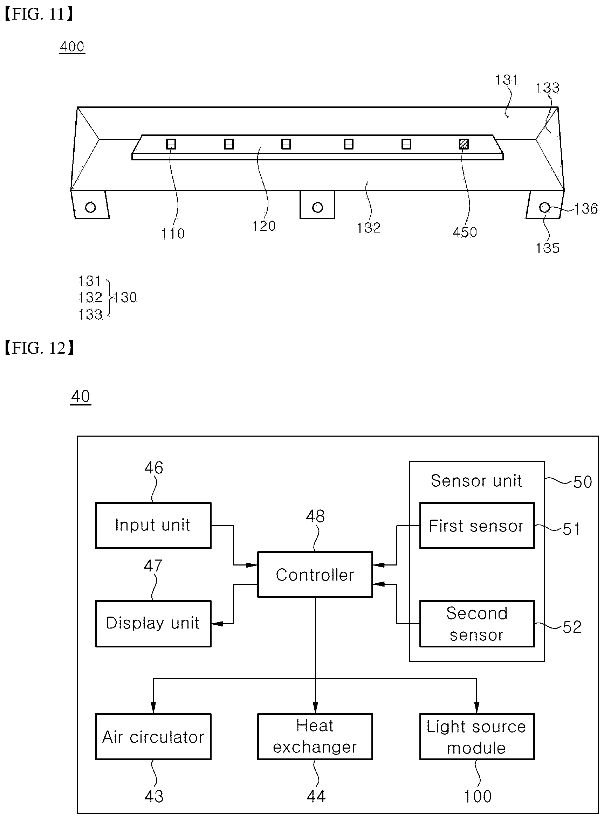

FIG. 11 is an exemplary view of a light source module according to a fourth embodiment of the present disclosure.

Referring to FIG. 11, a light source module 400 according to the fourth embodiment may include light sources 110, a substrate 120, a housing 130, and a sensor unit 450.

For description of the light sources 110, the substrate 120 and the housing 130 of the light source module 400 according to this embodiment, refer to description of the light sources 110, the substrate 120 and the housing 130 of the light source module 100 (see FIG. 1) according to the first embodiment.

According to this embodiment, the sensor unit 450 is mounted on the substrate 120.

The sensor unit 450 mounted on the substrate 120 may measure a concentration of pollutants in air passing through a sterilization area.

For example, the sensor unit 450 may include a photo-sensor adapted to measure a level of air pollution by receiving light reflected from pollutants in the air. Here, the light may be sterilizing light or may be light emitted from a light source 110 in the sensor unit 450.

In addition, the sensor unit 450 may include any type of sensor capable of measuring a pollution level of air as well as the photo-sensor.

The sensor unit 450 may measure the concentration of pollutants in air and may compare a measured result with a preset reference. Here, the reference value may be a reference of an air pollution level for determining whether air is polluted.

For example, when the concentration of pollutants is greater than or equal to the reference value, the sensor unit 450 may generate and send a light quantity increase signal to the light source module 400 or other components adapted to control operation of the light source module 400. Then, the light source module 400 may emit a greater quantity of sterilizing light in response to the light quantity increase signal sent from the sensor unit 450. For example, the quantity of light emitted from the light source module 400 may be increased by increasing electric current supplied to the light source module 400 or the number of light sources 110 operated to emit sterilizing light.

In addition, when the concentration of pollutants is less than the reference value, the sensor unit 450 may not generate a separate signal or may generate and send a light quantity decrease signal to the light source module 400 or the other components adapted to control operation of the light source module 400.

Then, the light source module 400 may emit a smaller quantity of sterilizing light in response to the light quantity decrease signal sent from the sensor unit 450. For example, the quantity of light emitted from the light source module 400 may be decreased by decreasing electric current supplied to the light source module 400 or the number of light sources 110 operated to emit sterilizing light.

The sensor unit 450 may include a sensor adapted to detect flow of external air in the light source module 400.

For example, the sensor unit 450 may be a sensor that detects an air volume or a flow rate of air flowing through a region in which the light source module 400 is mounted or through a sterilization area. The sensor unit 450 may generate a sterilization start signal when the measured air volume or flow rate is greater than or equal to a preset reference value. Here, the reference value may be an air volume value or a flow rate value serving as a reference for determining whether a device provided with the light source module 400 operates. In addition, the sensor unit 450 may generate a sterilization stop signal when the measured air volume or flow rate is less than the preset reference value.

Alternatively, the sensor unit 450 may detect operation of other components of the device provided with the light source module 400. For example, the sensor unit 450 may be electrically connected to a component included in the device to detect operation of the device by detecting whether electric power is supplied to the component of the device.

Upon detecting that electric power is supplied to the component of the device, the sensor unit 450 may generate the sterilization start signal. In addition, upon detecting that power supply to the component of the device is stopped, the sensor unit 450 may generate a sterilization stop signal.

In response to the sterilization start signal sent from the sensor unit 450, the light source module 400 may operate to emit sterilizing light. In addition, the light source module 400 may stop operation so as not to emit the sterilizing light in response to the sterilization stop signal sent from the sensor unit 450.

FIG. 12 is a block diagram of another embodiment of the air conditioner including the light source module according to the embodiment of the present disclosure.

Referring to FIG. 12, an air conditioner 40 according to this embodiment may include an input unit 46, a display unit 47, a controller 48, an air circulator 43, a heat exchanger 44, a light source module 100, and a sensor unit 50. Although the light source module 100 according to the first embodiment (see FIG. 1) is illustrated as the light source module 100 in FIG. 12, the light source module 100 may be selected from light source modules according to the other embodiments.

The input unit 46 may generate an air conditioning start signal to operate the air conditioner 40 or an air conditioning stop signal to stop operation of the air conditioner 40 in response to a manipulation signal input from outside.

Then, the input unit 46 may send the air conditioning start signal or the air conditioning stop signal to the controller 48. The air conditioning start signal may include information on the type of air conditioning and information on an air conditioning time. Here, the information on the type of air conditioning may include information on cooling, heating, ventilation, and the like.

The sensor unit 50 may include a first sensor 51 and a second sensor 52 that have different functions. The sensor unit 50 may be placed at any location so long as the sensor unit 50 can obtain information regarding air inside the air conditioner 40. In addition, the first sensor 51 and the second sensor 52 may be disposed at different locations. Further, the sensor unit 50 may be disposed on the substrate 120 as in the light source module 400 (see FIG. 11) according to the fourth embodiment.

The first sensor 51 may include a sensor adapted to detect the flow of air inside the air conditioner 40. For example, the first sensor 51 may detect the volume or flow rate of the air inside the air conditioner 40.

The first sensor 51 may generate a sterilization start signal when the measured volume or flow rate of the air inside the air conditioner 40 is greater than or equal to a preset reference value. Here, the reference value may be an air volume value or a flow rate value serving as a reference for determining whether the air conditioner 40 operates. In addition, the sensor unit 450 may generate a sterilization stop signal when the measured volume or flow rate of the air is less than the preset reference value. In addition, the first sensor 51 may generate a sterilization stop signal when the measured volume or flow rate of the air inside the air conditioner 40 is less than the preset reference value.

Alternatively, the first sensor 51 may detect operation of the air circulator 43. For example, the first sensor 51 may be electrically connected to the air circulator 43 to detect operation of the air circulator 43 by detecting whether electric power is supplied to the air circulator 43.

Upon detecting that electric power is supplied to the air circulator 43, the first sensor 51 may generate a sterilization start signal. In addition, upon detecting that power supply to the air circulator 43 is stopped, the first sensor 51 may generate a sterilization stop signal.

Then, the first sensor 51 may send the sterilization start signal or the sterilization stop signal to the controller 48.

According to this embodiment, the air conditioner 40 may detect operation of the air conditioner 40 through the first sensor 51 to allow sterilization to be automatically performed upon operation of the air conditioner 40.

The second sensor 52 may measure a pollution level of air passing through the sterilization area.

The second sensor 52 may measure the concentration of pollutants in air and may compare a measured result with a preset reference value.

When the measured value is greater than or equal to the reference value, the second sensor 52 may generate a light quantity increase signal.

Further, when the measured value is less than the reference value, the second sensor 52 may generate a light quantity decrease signal.

Then, the second sensor 52 may send the light quantity increase signal or the light quantity decrease signal to the sensor unit 50.

According to this embodiment, the air conditioner 40 may improve sterilization efficiency while preventing power loss by adjusting the quantity of the sterilizing light according to the degree of air pollution through the second sensor 52.

The controller 48 may control operation of each of components constituting the air conditioner 40 in response to signals sent from the input unit 46, the first sensor 51 and the second sensor 52.

In response to the air conditioning start signal sent from the input unit 46, the controller 48 may operate the air circulator 43 according to information in the air conditioning start signal. In addition, the controller 48 may operate the heat exchanger 44 according to information in the air conditioning start signal.

In response to the sterilization start signal sent from the first sensor 51, the controller 48 may allow power supply to the light source module 100 such that the light source module 100 can emit sterilizing light. Further, in response to the sterilization stop signal sent from the first sensor 51, the controller 48 may stop power supply to the light source module 100 to stop emission of the sterilizing light.

In response to the light quantity increase signal sent from the second sensor, the controller 48 may control operation of the light source module 100 to increase the quantity of the sterilizing light. For example, the controller 48 may increase the quantity of the sterilizing light by increasing the quantity of electric current supplied to the light source module 100. Alternatively, the controller 48 may increase the quantity of the sterilizing light by increasing the number of light sources 110 operated to emit the sterilizing light.

In response to the light quantity decrease signal sent from the second sensor 52, the controller 48 may control operation of the light source module 100 to decrease the quantity of the sterilizing light. For example, the controller 48 may decrease the quantity of the sterilizing light by decreasing the quantity of electric current supplied to the light source module 100. Alternatively, the controller 48 may decrease the quantity of the sterilizing light by decreasing the number of light sources 110 operated to emit the sterilizing light.

As such, according to this embodiment, the air conditioner 40 may perform automatic sterilization of air through the sensor unit while the air conditioning operation is performed, and may automatically change the quantity of the sterilizing light delivered to the air according to the degree of air pollution. That is, the air conditioner 40 according to this embodiment may employ the sensor unit to control the sterilization operation of the light source module in various ways.

Although some embodiments have been described herein with reference to the accompanying drawings, the above embodiments are provided by way of example. Therefore, it should be understood that the scope of the present disclosure is not limited to the above embodiments and should be defined only by the accompanying claims and equivalents thereto.

## Claims

1. A light source module comprising:
at least one light source emitting sterilizing light in a wavelength band capable of sterilizing pollutants;
a substrate on which the light source is mounted, the substrate being electrically connected to the light source; and
a light guide guiding a traveling direction of the sterilizing light emitted from the light source,
wherein the light guide guides the sterilizing light such that a beam angle of the light source module becomes narrower than a beam angle of the light source.

2. The light source module according to claim 1, wherein the light guide comprises a first housing and a second housing connected to each other,
the first housing being connected to the second housing to form a right angle or an acute angle therebetween.

3. The light source module according to claim 2, wherein the light source is disposed on an inner surface of the housing,
the inner surface of the housing being one surface of the first housing and one surface of the second housing connected to each other to form a right angle or an acute angle therebetween.

4. The light source module according to claim 3, wherein the sterilizing light is reflected from the one surface of the first housing and the one surface of the second housing.

5. The light source module according to claim 2, wherein a width of the first housing is different from a width of the second housing,
the width of the first housing being a length from one end of the first housing connected to the second housing to the other end thereof,
the width of the second housing being a length from one end of the second housing connected to the first housing to the other end thereof.

6. The light source module according to claim 1, wherein the light guide comprises:
a lens portion disposed on the light source and covering the light source; and
a cover portion connected to the lens portion and covering surroundings of the light source.

7. The light source module according to claim 6, wherein the light guide is formed of a silicone material allowing transmission of the sterilizing light therethrough.

8. The light source module according to claim 6, further comprising:
a housing on which the light source and the light guide are mounted,
the housing comprising a first housing and a second housing connected to each other,
the first housing being connected to the second housing to form a right angle or an acute angle therebetween.

9. The light source module according to claim 8, wherein the light source is disposed on an outer surface of the housing,
the outer surface of the housing being an opposite surface to an inner surface of the housing, and
the inner surface of the housing is one surface of the first housing and one surface of the second housing connected to each other to form a right angle or an acute angle therebetween.

10. The light source module according to claim 1, wherein:
the light guide comprises a cavity open at an upper side thereof;
an inner wall of the light guide defining the cavity is an inclined surface; and
the light source is mounted on the cavity.

11. The light source module according to claim 10, further comprising:
a sealing portion sealing the cavity of the light guide and formed of a material allowing transmission of the sterilizing light therethrough.

12. The light source module according to claim 10, further comprising:
a housing on which the light source and the light guide are mounted,
the housing comprising a first housing and a second housing connected to each other,
the first housing being connected to the second housing to form a right angle or an acute angle therebetween.

13. The light source module according to claim 12, wherein the light source is disposed on an outer surface of the housing,
the outer surface of the housing being an opposite surface to an inner surface of the housing, and
the inner surface of the housing is one surface of the first housing and one surface of the second housing connected to each other to form a right angle or an acute angle therebetween.

14. The light source module according to claim 1, further comprising:
a sensor unit detecting a pollution level of a sterilization target or detecting a timing of emitting sterilizing light.

15. An air conditioner comprising:
an air inlet through which air is introduced into the air conditioner;
an air outlet through which the air is discharged from the air conditioner;
an air circulator guiding the air to flow in a direction from the air inlet to the air outlet;
a heat exchanger performing heat exchange with the air; and
at least one light source module emitting sterilizing light in a wavelength band capable of sterilizing pollutants towards a sterilization area between the air inlet and the heat exchanger, the light source module comprising:
at least one light source emitting the sterilizing light;
a substrate on which the light source is mounted, the substrate being electrically connected to the light source; and
a light guide guiding a traveling direction of the sterilizing light emitted from the light source,
wherein the light guide guides the sterilizing light to be delivered to the sterilization area such that a beam angle of the light source module becomes narrower than a beam angle of the light source.

16. The air conditioner according to claim 15, wherein the light guide comprises a first housing and a second housing connected to each other,
the first housing being connected to the second housing to form a right angle or an acute angle therebetween.

17. The air conditioner according to claim 16, wherein the light source is disposed on an inner surface of the housing,
the inner surface of the housing being one surface of the first housing and one surface of the second housing connected to each other to form a right angle or an acute angle therebetween.

18. The air conditioner according to claim 17, wherein the sterilizing light is reflected from the one surface of the first housing and the one surface of the second housing.

19. The air conditioner according to claim 16, wherein a width of the first housing is different from a width of the second housing,
the width of the first housing being a length from one end of the first housing connected to the second housing to the other end thereof,
the width of the second housing being a length from one end of the second housing connected to the first housing to the other end thereof.

20. The air conditioner according to claim 15, wherein the light guide comprises:
a lens portion disposed on the light source and covering the light source; and
a cover portion connected to the lens portion and covering surroundings of the light source.

21. The air conditioner according to claim 20, wherein the light guide is formed of a silicone material allowing transmission of the sterilizing light therethrough.

22. The air conditioner according to claim 20, further comprising:
a housing on which the light source and the light guide are mounted,
the housing comprising a first housing and a second housing connected to each other,
the first housing being connected to the second housing to form a right angle or an acute angle therebetween.

23. The air conditioner according to claim 22, wherein the light source is disposed on an outer surface of the housing,
the outer surface of the housing being an opposite surface to an inner surface of the housing, and
the inner surface of the housing is one surface of the first housing and one surface of the second housing connected to each other to form a right angle or an acute angle therebetween.

24. The air conditioner according to claim 15, wherein:
the light guide comprises a cavity open at an upper side thereof;
an inner wall of the light guide defining the cavity is an inclined surface; and
the light source is mounted on the cavity.

25. The air conditioner according to claim 24, further comprising:
a sealing portion sealing the cavity of the light guide and formed of a material allowing transmission of the sterilizing light therethrough.

26. The air conditioner according to claim 24, further comprising:
a housing on which the light source and the light guide are mounted,
the housing comprising a first housing and a second housing connected to each other,
the first housing being connected to the second housing to form a right angle or an acute angle therebetween.

27. The air conditioner according to claim 26, wherein the light source is disposed on an outer surface of the housing,
the outer surface of the housing being an opposite surface to an inner surface of the housing, and
the inner surface of the housing is one surface of the first housing and one surface of the second housing connected to each other to form a right angle or an acute angle therebetween.

28. The air conditioner according to claim 25, wherein:
the air circulator is disposed between the air inlet and the heat exchanger;
the sterilization area is a region between the air inlet and the air circulator through which the air passes; and
the light source module is disposed outside the sterilization area to be closer to the air circulator than to the air inlet.

29. The air conditioner according to claim 28, wherein the air inlet and the air circulator are not exposed to the sterilizing light emitted from the light source module.

30. The air conditioner according to claim 15, wherein the heat exchanger is disposed between the air inlet and the air circulator, and the sterilization area is a region between the air inlet and the heat exchanger through which the air passes.

31. The air conditioner according to claim 30, wherein:
the heat exchanger is an evaporator;
the light source module emits the sterilizing light towards the sterilization area and the evaporator; and
the air circulator is not exposed to the sterilizing light.

32. The air conditioner according to claim 15, further comprising:
a sensor unit detecting a pollution level of a sterilization target or detecting a timing of emitting sterilizing light.
